# EUROPEAN PATENT SPECIFICATION

(11) **EP 0 565 897 B1**
(45) Date of publication and mention of the grant of the patent: **26.06.2002**
(21) Application number: 93104458.0
(22) Date of filing: 18.03.1993
(51) Int. Cl.: A61K 31/47

(54) **Argatroban preparations for ophthalmic use**
Zusammensetzung von Argatroban zur Verwendung in Ophthalmologie
Composés de l'argatroban pour utilisation ophthalmologique

(30) Priority: 18.03.1992 JP 6194792
(43) Date of publication of application: 20.10.1993
(73) Proprietor: MITSUBISHI CHEMICAL CORPORATION, Chiyoda-ku Tokyo (JP)
(72) Inventor: Mano, Tomiya, Ibaraki-shi, Osaka-fu (JP); Shiomura, Jin, Funabashi-shi, Chiba-ken (JP)
(74) Representative: Hansen, Bernd, Dr. Dipl.-Chem.

(56) References cited:
- THROMB. RES. vol. 45, no. 5, 1987, pages 699 - 702 A. HIJIKATA-OKUNOMIYA ET AL 'Effect of MD-805 on plasminogen activator release by thrombin from isolated perfused dog leg.'
- THROMB. RES. vol. 64, no. 6, 1991, pages 677 - 689 J. SCHNEIDER 'Heparin and the thrombin inhibitor argatroban enhance fibrinolysis by infused or bolus-injected saruplase (R-SCU-PA) in rabbit femoral artery thrombosis.'
- OPHTHALMOLOGY vol. 97, no. 2, 1990, pages 184 - 189 G.J. JAFFE ET AL 'Tissue plasminogen activator for postvitrectomy fibrigin formation.'

## Description

### INDUSTRIAL FIELD OF INVENTION

This invention relates to a novel use of argatroban in the field of ophthalmology. More specifically, it relates to the novel use of argatroban for inhibiting fibrin formation in the anterior chamber.

### BACKGROUND OF THE INVENTION

Argatroban is a generic name designated to (2R,4R)-4-methyl-1-[N²-((RS)-3-methyl-1,2,3,4-tetrahydro-8-quinolinesulfonyl)-L-arginyl]-2-piperidinecarboxylic acid hydrate represented by the formula: 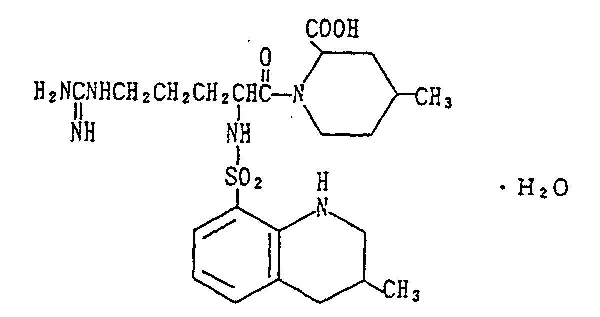 This compound belongs to N²-arylsulfonyl-L-arginineamides.

As is disclosed in Japanese Patent Application 88786/1979, argatroban is a selective anti-thrombin substance having entirely new action mechanism which has never been observed in hitherto known medicines. The action mechanism of argatroban includes the selective inhibition of thrombin caused by steric binding of the tripod structure of argatroban to the active site of thrombin. Argatroban strongly inhibits three major actions of thrombin, i.e., (1) fibrin formation, (2) stabilization of fibrin by activation of Factor VIII, and (3) platelet aggregation. As a result, argatroban is clinically known to be applicable in treating limbs ulcer in chronic arterial obstruction and pain at rest, and improving frigidity.

The present inventors completed this invention as explained hereunder in detail by searching for applications of argatroban other than in chronic arterial obstruction in the light of the above unique action mechanism of argatroban.

After the intraocular surgery such as retinal and vitreous surgery, cataract operation, and glaucoma operation, the post operative fibrin formation in anterior chamber is often observed. This is a very important problem since the fibrin formation must be prevented for establishing satisfactory post operative management. For example, the fibrin formation after intraocular lens implantation results in not only poor visual prognosis, but also possible development of serious condition, such as complication of glaucoma. The fibrin formation after vitreous surgery also interferes the post operative fundus examination to disturb the appropriate treatment or management of vitreo-retinal disease. Further, the fibrin formation can cause intractable anterior proliferative vitreo-retinal condition.

On the background as explained above, it has been recognized that prevention of postoperative fibrin formation is very important, but any measure to cope with it has never been reported to date.

### BRIEF EXPLANATION OF FIGURES

FIGURE 1 : Fibrin formation in the anterior chamber by the laser irradiation under condition A. This is a photograph replacing a figure showing the appearance of the animal.

FIGURE 2: Fibrin formation in the anterior chamber by the laser irradiation under condition B. This is a photograph replacing a figure showing the appearance of the animal.

FIGURE 3: Inhibition of fibrin formation caused by the laser irradiation under condition A in the anterior chamber with argatroban. This is a photograph replacing a figure showing the appearance of the animal.

FIGURE 4: Inhibition of fibrin formation caused by the laser irradiation under condition A in the anterior chamber with argatroban. This is a photograph replacing a figure showing the appearance of the animal.

FIGURE 5: Inhibition of fibrin formation caused by administering self-plasma in the anterior chamber with argatroban. This is a photograph replacing a figure showing the appearance of the animal.

FIGURE 6: Fibrin formation in the anterior chamber by caused by administering self-plasma. This is a photograph replacing a figure showing the appearance of the animal.

### EXPLANATION OF THIS INVENTION

Argatroban has been hitherto administered for preventing the formation of the blood clot in blood vessels, i.e., thrombus as disclosed in A. Hijikata-Okunomiya et al, Thromb, Res., vol 45, no 5, 1987 and in J. Schneider, Thromb, Res. vol. 64, no 6, 1991, p. 677-689.

The present inventors conducted a pharmacological experiment to see whether or not argatroban can show its unique in vivo activity in tissues or organs other than blood. It was found that argatroban can prevent the fibrin formation after intraocular surgery. The present invention is based on this finding. No report has been presented to confirm such activity of argatroban in vivo other than in blood. Argatroban has never been applied in the ophthalmic area yet.

In order to prevent the post-opertive fibrin formation in the eye, Argatroban can be administered, for example, by direct application to the anterior chamber, by application as an eye drop, by intravenous application, or by application in an intraocular irrigating solution, and the like methods. The intravenous application is preferably by the drip infusion. Further, argatroban can be injected under the retina. Argatroban can be administered prior to, during, or after the operation.

The pharmaceutical formulation used for administering argatroban is parenteral solution such as intraocular irrigating solution, eye drop, or drip infusion.

The intraocular irrigating solution used in this invention is prepared by dissolving argatroban in, for example, sterile and purified water. In this case, if necessary, pharmaceutically acceptable additives can be added such as a buffering agent and an isotonic agent for adjusting the composition of the solution to that of the aqueous humor. Specifically, glucose, sodium chloride, potassium chloride, calcium chloride, magnesium sulfate, sodium hydrogen carbonate, etc., can be added.

The eye drop used in this invention is an aqueous ophthalmic solution, non-aqueous ophthalmic solution, ophthalmic suspension, or opthalmic emulsion. The eye drop of this invention is prepared by dissolving or suspending argatroban in sterile purified water, physiological saline, etc., as the aqueous solvent, or cotton seed oil, soybean oil, sesame oil, peanut oil, and the like plant oil as the non-aqueous solvent. In this case, isotonic agent, pH adjusting agent, viscousifying agent, suspending agent, emulsifying agent, preserving agent, and the like pharmaceutically acceptable additives can be added, if necessary. Specifically, the isotonic agents include sodium chloride, boric acid, sodium nirate, potassium nitrate, D-mannitol, glucose, etc. Specific examples of the pH adjusting agents include boric acid, anhydrous sodium sulfite, hydrochloric acid, ciric acid, sodium citrate, acetic acid, potassium acetate, sodium carbonate, borax, etc. Specific examples of the viscosifying agents include methylcellulose, hydroxypropylmethylcellulose, polyvinyl alcohol, sodium condroitin sulfate, polyvinylpyrrolidone, etc. Specific examples of the suspending agents include polysolvate 80, polyoxyethylene hydrogenated caster oil 60, polyoxy hydrogenated caster oil, etc. Specific examples of the emulsifying agents include yolk lecithin, polysolvate 80, etc. Specific examples of the preserving agents include benzalkonium chloride, benzethonium chloride, chlorobutanol, phenylethyl alcohol, paraoxybenzoic acid esters, etc.

The drip infusion used in this invention is an aqueous solution for injection and a suspension for injection. The drip infusion of this invention is prepared by dissolving argatroban in distilled water for injection, physiological saline, Ringer solution, or in these solvents containing a small amount of water-soluble organic solvent such as ethanol, glycerine which are used as a solvent. In this case, isotonic agent, pH adjusting agent, emulsifying agent, preserving agent, and the like pharmaceutically acceptable additives can be added, if necessary. Specific examples of the isotonic agents include sodium chloride, glucose, D-sorbitol, D-mannitol, etc. The pH adjusting agents include anhydrous sodium sulfite, hydrochloric acid, citric acid, sodium citrate, etc. The emulsifying agents include yolk lecithin, polysolvate 80, etc. The preserving agents include benzalkonium chloride, benzethonium chloride, paraoxybenzoate esters, etc.

As is illustrated in the following examples, the administration of argatroban to anterior chamber together with self-plasma causes inhibition of fibrin formation. Such result shows that the fibrin formation may be inhibited by deliver of argatroban into the anterior chamber. This result can support that argatroban can be given by direct administration to the anterior chamber and, similarly, it is understood that the fibrin formation after intraocular operation can be inhibited by adding it into ocular infusion. The experiments on rabbits conducted by the present inventors to date showed that the argatroban concentration in the anterior chamber 1 hour after the intravenous administration of 10 microgram/kg/min, 40 microgram/kg/min, and 400 microgram/kg/min argatroban are 5.7 ± 0.7, 41.0 ± 4.2, and 881 ± 110 mg/ml, respectively. Further, administration of eye drops of 2 mg/ml argatroban in saline results in the concentration of 23.7 ± 19.1 mg/ml in the anterior chamber. This means that by intravenous and eye-drop administrations argatroban is delivered into the anterior chamber and the fibrin formation in the organ can be inhibited.

Optimal dose of argatroban for man may vary depending on the administration route, administration time, age of the patient, condition of patients, etc. Generally, applicable may be 1 to 50 mg/kg/day for intravenous administration, an ocular solution of 1 to 6 mg/ml argatroban for eye drop administration, and 0.1 microgram/ml to 0.6 mg/ml argatroban in intraocular irrigation solution.

### EXAMPLES

The inhibition effect of argatroban on fibrin formation in the anterior chamber is explained by detailed Examples as follows. Those skilled in the art can understand that novel treatment methods of this invention provide decrease of fibrin formation after cataract operation, vitreous surgery, and glaucoma operation so as to result in more satisfactory restoration of visual acuity.

### Example 1

### Fibrin formation in the anterior chamber caused by laser irradiation

### Experimental model

In this example, a model of fibrin formation in the rabbit anterior chamber caused by laser irradiation was used as a measure for testing the action of argatroban. In this model, the seriousness of inflammation and amount of fibrin are determined proportional to the total energy of the laser irradiation. The amount of fibrin can be controled to the desired value by altering total energy of laser irradiation. In this example, two conditions with smaller (condition A) and larger (conditionB) irradiation energy were used to test fibrin formation, finding that stronger fibrin formation was observed in the eyes treated with the larger total irradiation energy.

### MATERIALS AND METHOD

Matured pigmented rabbits (Dutch rabbits of 2 kg body weight) were irradiated with laser at the iris using an argon laser equipment (product of NIDEK). In one group of the rabbits on the eyes, 8 irradiation points at the same interval on a circle line were made under the conditions of coagulation size of 50 micrometer, output of 1.0 Watt and irradiation time of 0.1 second (condition A). In other group of the rabbits, 8 irradiation points similar to those on the eyes were made under the conditions of coagulation size of 200 micrometer, output of 1.0 Watt and irradiation time of 0.2 second (condition B).

From 30 minutes prior to and until 30 minutes after irradiations, 0.1 mg/kg/minute of argatroban was administered intravenously from ear vein. Control group received only laser irradiation. Positive control group received 1000 U/kg of heparin intravenously 30 minutes prior to laser irradiation.

For argatroban eye drop, right eyes of the rabbits received 5 mg/ml solution of argatroban, while left eyes were used as a control without application of argatroban.

The fibrin formation was recorded photographically using a slit lamp microscope.

Further, the laser irradiation of condition B was effected and then the anterior chamber flare values were estimated using the flare cell meter at 30 minutes and 60 minutes after the irradiation.

### RESULTS

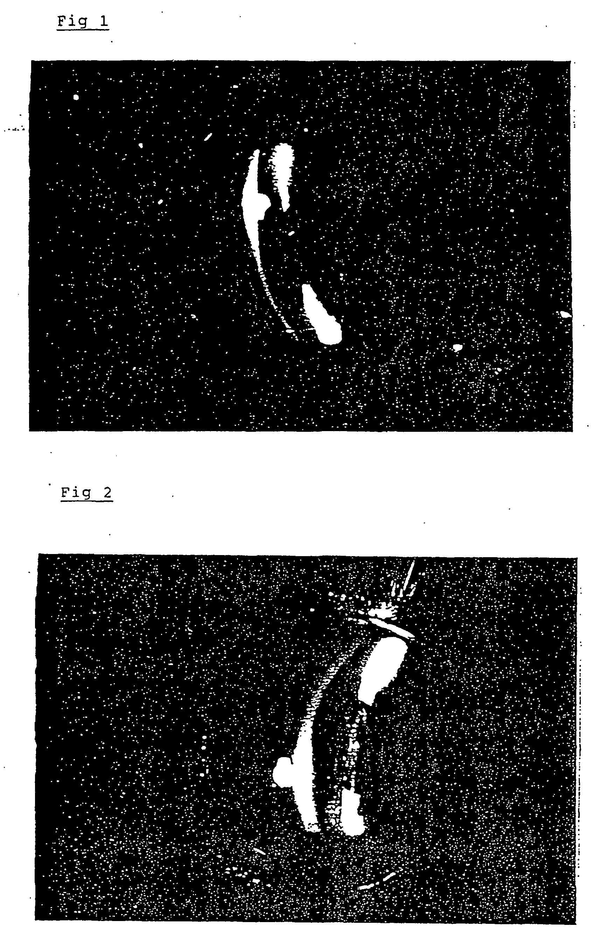

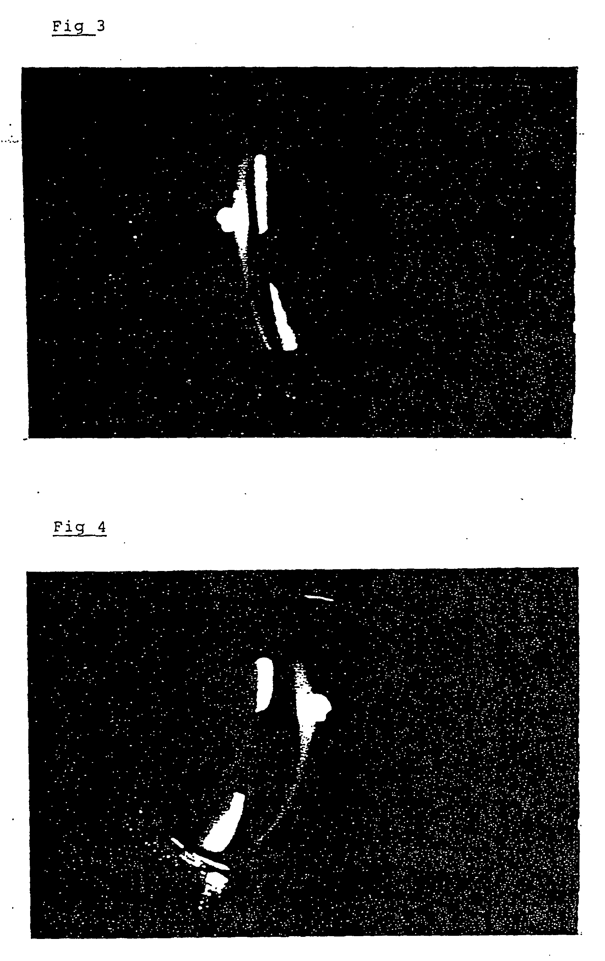

In the control group, fibrin formation were observed in both of conditions A and B after 30 minutes. The results obtained by a slit lamp microscope for conditions A and B are shown in Figures 1 and 2, respectively. In the positive control group (heparin administered group), no fibrin formation was observed during 30 minutes to 3 hours observation time after the irradiation (The result is not shown). In the 0.1 mg/kg/minute argatroban administered group, no fibrin formation was also observed 30 minutes after the irradiation under both of conditions A and B, similar to the heparin administered group. The results under conditions A and B are shown in Figures 3 and 2 as attached.

The results of the flare cell meter observations are shown in Table 1 below.

The flare cell meter measures turbidity in the anterior chamber as the photon count. The following Table 1 shows that argatroban clearly inhibited the fibrin formation after 30 minutes and 60 minutes. This experiment also showed the inhibiting activity of argatroban against fibrin formation in the anterior chamber.

**TABLE 1**

| The effect of argatroban on flare increase in the anterior chamber of pigmented rabbits after the irradiation of argon laser | | |
|---|---|---|
| Medical | photon count (/msec) | |
| | 30 minutes after laser irradiation | 60 minutes after laser irradiation |
| control | 877.0 ± 333.8 (n=4) | 1002.9 ± 137.0 (n=4) |
| argatroban i.v. | 417.7 ± 159.3 (n=4) | 356.9 ± 168.3 (n=4) |
| argatroban eye drop | 287.7 ± 99.2 (n=4) | 341.2 ± 86.5 (n=4) |
| Note: n = number of animals. argatropan i.v. = intravenous injection at 0.1 mg/kg/min. argatroban eye drop = a solution of 5 mg/ml concentration was used. | | |

### Example 2

### Fibrin formation by administering self-plasma to the anterior chamber

### Experimental model

Blood of matured white rabbits (2 kg in body weight) were taken and separated to give plasma. Two hundreds microliter of anterior chamber liquid was taken by conducting centesis into anterior chamber of the same rabbit. Then, 150 microliter of the plasma obained and 50 microliter of argatroban solution (10 mg/ml), 200 microliter in total, were injected to the anterior chamber from the same centesis portion. The control received 150 microliter of plasma and 50 microliter of physiological saline in the same manner at the anterior chamber. The fibrin formation in the anterior chamber 24 hours after the administration was observed and recorded.

### RESULTS

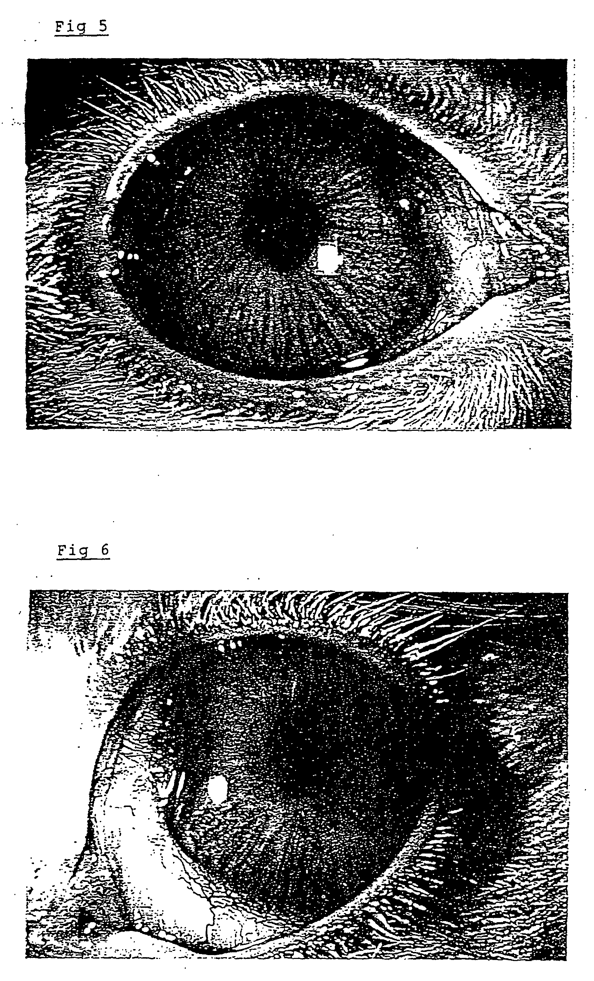

No fibrin formation was observed 24 hours after the administration of arbatroban. On the contrary, a strong fibrin formation in the anterior chamber was observed in the control eyes. The obtained results are shown in Figures 5 and 6 as attached.

### DISCUSSION

As is apparent from Examples 1 and 2 as described above, intravenous administration of argatroban provides complete inhibition of the fibrin formation in the anterior chamber in both of laser irradiation model and self-plasma injection model. This should be a result of competition of argatroban with the action of trombin during the course of conversion of fibrinogen to fibrin.

Argatroban will prevent the fibrin formation and blood clot formation by direct action on trombin, and it will not work on other coagulation systems. This means that less risk of bleeding after intraocular operation is expected as compared with other anticoagulating agents, e.g., heparin. If the argatroban administration ceases, the coagulating function of the body is recovered rapidly. It seems that argatroban is an extremely effective and safe drug for application in clinical ophthalmology.

In the following Table 2, the toxicological data of argatroban is shown.

**TABLE 2**

| LD50 of argatroban (mg/kg) | | | | | |
|---|---|---|---|---|---|
| animal | sex | i. v. | i. p. | subc. | p. o. |
| mouse | Male | > 81 | 475 | 3750 | > 15000 |
| | Female | > 81 | 640 | 3900 | > 15000 |
| rat | Male | > 81 | 320 | 700 | > 15000 |
| | Female | > 81 | 409 | 620 | > 15000 |
| dog | Male | > 200 | - | - | - |
| | Female | > 200 | - | - | - |

## Claims

1. Use of argatroban represented by the formula: 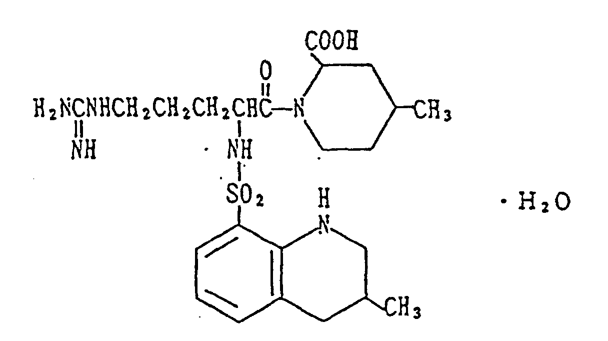 in the preparation of a medicament for inhibiting fibrin formation in the anterior chamber of the eye in mammals.

2. The use as claimed in claim 1, wherein the medicament is in the form of an ocular irrigation.

3. The use as claimed in claim 1, wherein the medicament is in the form of an eye drop.

4. The use as claimed in claim 1, wherein the medicament is in the form of a drip infusion.

5. The use of argatroban represented by the formula: 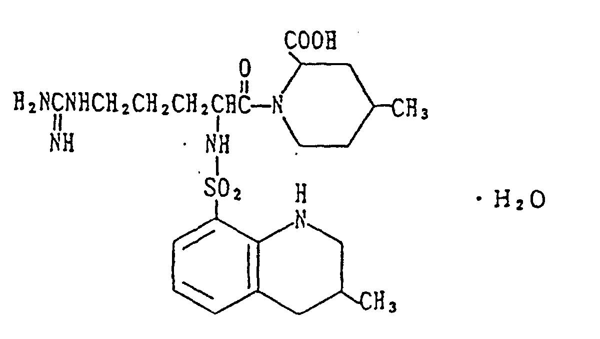 in the manufacture of a medicament for the prophylaxis of post-operative fibrin formation in the anterior chamber of the eye of a mammal.

6. The use according to claim 1 or claim 5, wherein the medicament is directly administrable into the anterior chamber.

7. The use according to claim 5, wherein the medicament is in the form of an irrigating solution, in the form of an eye drop or in the form of a drip infusion.

8. The use according to claim 5, wherein the medicament is administrable by intravenous injection.

## Patentansprüche

1. Verwendung von Argatroban, dargestellt durch die Formel: 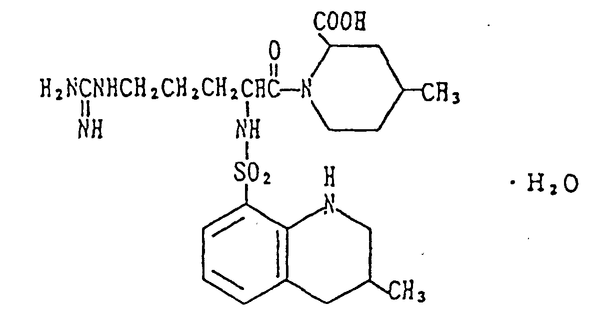 in der Herstellung eines Medikaments zur Hemmung der Fibrinbildung in der vorderen Augenkammer bei Säugetieren.

2. Verwendung gemäß Anspruch 1, worin das Medikament in der Form einer Augenspülung ist.

3. Verwendung gemäß Anspruch 1, worin das Medikament in der Form von Augentropfen ist.

4. Verwendung gemäß Anspruch 1, worin das Medikament in der Form einer Tropfinfusion ist.

5. Verwendung von Argatroban, dargestellt durch die Formel: 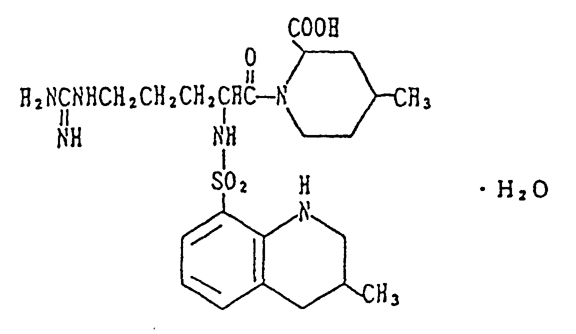 in der Herstellung eines Medikaments zur Prophylaxe von postoperativer Fibrinbildung in der vorderen Augenkammer eines Säugetiers.

6. Verwendung gemäß Anspruch 1 oder 5, worin das Medikament direkt in die vordere Augenkammer verabreichbar ist.

7. Verwendung gemäß Anspruch 5, worin das Medikament in der Form einer Spüllösung, in der Form von Augentropfen oder in der Form einer Tropfinfusion ist.

8. Verwendung gemäß Anspruch 5, worin das Medikament durch intravenöse Injektion verabreichbar ist.

## Revendications

1. Utilisation d'argatroban représenté par la formule : 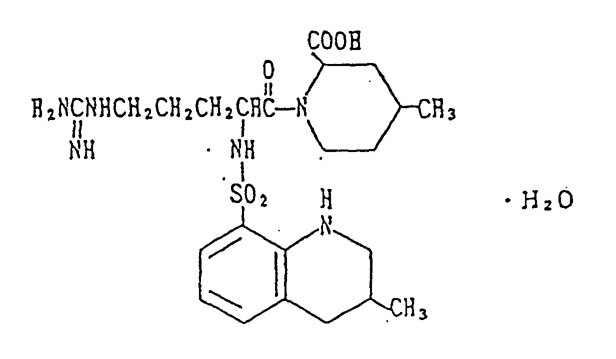 dans la préparation d'un médicament destiné à inhiber la formation de fibrine dans la chambre antérieure de l'oeil chez les mammifères.

2. Utilisation selon la revendication 1, dans laquelle le médicament se trouve sous forme d'une irrigation oculaire.

3. Utilisation selon la revendication 1, dans laquelle le médicament se trouve sous forme d'un collyre.

4. Utilisation selon la revendication 1, dans laquelle le médicament se trouve sous forme d'une perfusion.

5. Utilisation d'argatroban représenté par la formule 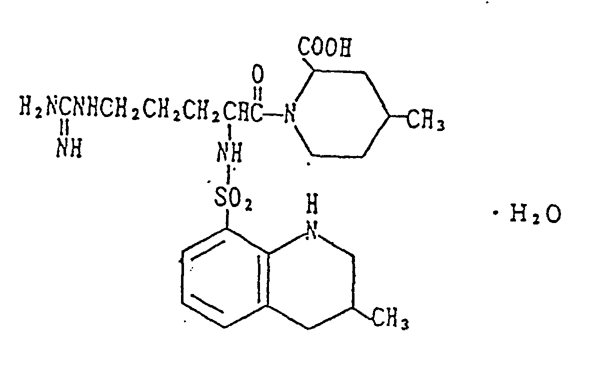 dans la fabrication d'un médicament destiné à la prophylaxie de la formation post-opératoire de fibrine dans la chambre antérieure de l'oeil d'un mammifère.

6. Utilisation selon la revendication 1 ou la revendication 5, dans laquelle le médicament est susceptible d'être administré directement dans la chambre antérieure.

7. Utilisation selon la revendication 5, dans laquelle le médicament se trouve sous forme d'une irrigation oculaire, sous forme d'un collyre ou sous forme d'une perfusion.

8. Utilisation selon la revendication 5, dans laquelle le médicament est susceptible d'être administré par injection intraveineuse.
